# DEMANDE DE BREVET EUROPEEN

(11) **EP 3 269 397 A1**
(43) Date de publication de la demande: **17.01.2018**
(21) Numéro de dépôt: 17180670.6
(22) Date de dépôt: 11.07.2017
(51) Int. Cl.: A61L 9/03, A01M 13/00, A61M 11/04, A61M 15/00, C11B 9/00, B01D 11/00

(54) **DIFFUSEUR DE VAPEURS, NOTAMMENT D'HUILES ESSENTIELLES**

(30) Priorité: 13.07.2016 FR 1656750
(71) Demandeur: Allie, Guillaume, 44590 Derval (FR)
(72) Inventeur: Allie, Guillaume, 44590 Derval (FR)
(74) Mandataire: Nony

(57) **Abrégé**

Diffuseur (1) de vapeurs, notamment d'huiles essentielles, comportant :
- un corps creux (2),
- un bac (6) pour contenir au moins un liquide (L) et éventuellement des huiles essentielles (H), logé dans une partie inférieure (4) du corps creux (2),
- un système de chauffage (10) pour chauffer le liquide (L) présent dans le bac afin de créer des vapeurs (V),
- au moins un orifice de sortie (16) des vapeurs réalisé dans une partie supérieure (3) du corps creux (2),
- un dispositif de régulation du débit de vapeurs (20) s'échappant par l'orifice de sortie (16), disposé dans le corps creux (2) entre le bac (6) et l'orifice de sortie (16), et
- un organe de commande (25) dudit dispositif de régulation du débit de vapeurs (20).

## Description

### Domaine de l'invention

La présente invention concerne le domaine des diffuseurs de vapeurs, notamment d'huiles essentielles. L'invention concerne plus particulièrement un diffuseur de vapeurs d'huiles essentielles, notamment les diffuseurs dits par chaleur douce ainsi qu'un procédé de diffusion de vapeurs d'huiles essentielles à l'aide d'un tel diffuseur, un procédé de réalisation d'une infusion et/ou d'une décoction et/ou d'une macération à l'aide d'un tel diffuseur et un procédé de réalisation d'un liquide à destination d'une inhalation à l'aide d'un tel diffuseur.

### Art antérieur et objectifs de l'invention

On connaît à ce jour quatre types de diffusion des huiles essentielles, à savoir la diffusion par brumisation, la diffusion par chaleur douce, la diffusion par nébulisation et la diffusion par ventilation.

La diffusion par brumisation, encore appelée diffusion à ultrasons, fonctionne à l'aide d'ondes ultrasoniques de très haute fréquence produites par un diffuseur, qui permettent de transformer un mélange d'eau froide et d'huiles essentielles en une légère brume chargée d'huiles essentielles. Ce type de diffuseur présente l'avantage d'avoir un dégagement de brume, ce qui procure un effet esthétique. De plus, la diffusion se fait à froid, sans risque d'altération des huiles essentielles. Grâce à un tel diffuseur, l'air ambiant est moins sec. Le rapport qualité-prix d'un tel diffuseur est intéressant. Cependant, les diffuseurs fonctionnant par brumisation peuvent être peu silencieux, à cause du bruit du moteur et du clapotement de l'eau dû aux gouttelettes retombant dans le réceptacle. De plus, les particules d'huiles essentielles, gorgées d'eau, ne restent pas longtemps en suspension dans l'air. Enfin, la durée de vie de tels diffuseurs est peu satisfaisante.

La diffusion par nébulisation, encore appelée diffusion par micro-diffusion, consiste à produire des vibrations qui, au contact des huiles essentielles placées au sein d'un réceptacle en verre de forme spécifique, projettent un nuage de fines particules d'huiles essentielles. Grâce à la légèreté des particules d'huiles essentielles, les diffuseurs par nébulisation peuvent diffuser dans de grands espaces pouvant aller jusqu'à 120 m². La diffusion à froid permet d'empêcher l'altération des huiles essentielles. La diffusion à sec permet de ne pas nécessiter d'eau. Généralement, de tels diffuseurs sont esthétiques et ont une bonne durée de vie. Cependant, ils sont peu silencieux à cause de la présence d'une pompe à air. De plus, ils nécessitent une quantité importante d'huiles essentielles, sont relativement fragiles, sont relativement difficiles d'entretien et sont coûteux.

En ce qui concerne les diffuseurs par ventilation, ceux-ci fonctionnent à l'aide d'un souffle d'air qui passe sur un buvard jetable imprégné d'huiles essentielles et qui propulse des particules d'huiles essentielles dans l'air. La diffusion se fait à froid, sans risque d'altération des huiles essentielles, et à sec, ne nécessitant pas d'eau. De tels diffuseurs sont généralement robustes et relativement peu coûteux avec une technologie adaptée pour des formes transportables de diffuseurs. Cependant, ces diffuseurs sont relativement bruyants à cause du ventilateur. De plus, la puissance est généralement faible avec un volume de diffusion très limité, certaines huiles essentielles peuvent être mal diffusées et ainsi le diffuseur n'est pas adapté pour une pièce. Enfin, un tel diffuseur nécessite un consommable, le buvard.

Par ailleurs, les diffuseurs par brumisation, par nébulisation et par ventilation ne permettent pas à tout moment de maîtriser avec précision la puissance de diffusion des huiles essentielles. Leur fonctionnement à moteur, avec pompe à air ou ventilateur, peut être bruyant et inconfortable. La diffusion des huiles essentielles et le bruit de ces diffuseurs peuvent véritablement être ressentis comme agressifs pour les voies respiratoires ou les oreilles. Par ailleurs, ces diffuseurs utilisent comme source d'énergie un transformateur qui est davantage consommateur d'électricité qu'un branchement électrique classique, et même lorsque ces diffuseurs sont mis hors tension, certains composants du transformateur continueront à consommer un peu d'électricité.

Les diffuseurs par chaleur douce fonctionnent à l'aide d'un chauffage doux, en général à 45 °C, d'un mélange eau et huiles essentielles. Sous l'effet de cette chaleur régulée, on produit des particules d'huiles essentielles. De tels diffuseurs sont simples d'utilisation, robustes, faciles d'entretien, peu coûteux, ne nécessitant que quelques gouttes d'huiles essentielles et sont totalement silencieux. Cependant, le volume de diffusion est relativement limité, pouvant convenir pour une pièce de taille moyenne. Un autre inconvénient de tels diffuseurs est de ne pouvoir garantir une sécurité optimale lors du nettoyage du bac, avec sa partie électrique, contenant l'eau et les huiles essentielles.

Il existe ainsi un besoin pour bénéficier d'un diffuseur silencieux permettant de bien conserver la qualité des principes actifs des huiles essentielles.

Il existe encore un besoin pour disposer d'un diffuseur d'huiles essentielles, notamment par chaleur douce, qui soit optimal en termes de qualité, d'efficacité et de sécurité.

Il existe également un besoin pour bénéficier d'un dispositif permettant la préparation d'autres thérapeutiques naturelles.

### Résumé de l'invention

La présente invention a ainsi pour objet, selon un premier de ses aspects, un diffuseur de vapeurs, notamment d'huiles essentielles, de préférence par chaleur douce comportant :
- un corps creux,
- un bac pour contenir au moins un liquide et éventuellement des huiles essentielles, logé dans une partie inférieure du corps creux,
- un système de chauffage pour chauffer le liquide présent dans le bac afin de créer des vapeurs,
- au moins un orifice de sortie des vapeurs réalisé dans une partie supérieure du corps creux,
- un dispositif de régulation du débit de vapeurs s'échappant par l'orifice de sortie, disposé dans le corps creux entre le bac et l'orifice de sortie, et
- un organe de commande dudit dispositif de régulation du débit de vapeurs. Grâce à l'invention, mettant avantageusement en oeuvre le chauffage par chaleur douce, la quintessence des huiles essentielles est préservée. En effet, la diffusion par chaleur douce peut être définie comme l'évaporation des vapeurs, notamment d'huiles essentielles, à l'aide d'une source de chaleur, constituée par le système de chauffage, à température modérée.

Grâce à la présence du dispositif de régulation du débit de vapeurs et à son organe de commande, la puissance de diffusion peut être contrôlée.

Dans l'ensemble de la description, l'expression «huiles essentielles» est au pluriel, mais cela n'exclut aucunement la possibilité de n'avoir qu'une seule huile essentielle.

Le corps creux présente avantageusement au moins partiellement une forme allongée selon un axe sensiblement vertical, par exemple sensiblement une forme de colonne verticale. Le corps creux peut présenter une forme générale de S. Le corps creux peut présenter une hauteur maximale comprise entre 30 cm et 60 cm, et un diamètre maximal selon un axe horizontal compris entre 10 cm et 20 cm. Les expressions « bas » et « haut », « partie inférieure » et « partie supérieure » sont comprises au sens de l'invention en référence au diffuseur posé sur un support. Le bas et la partie inférieure sont les plus proches du support, tandis que le haut et la partie supérieure sont les plus éloignés du support, le regard d'un observateur pour passer du bas et de la partie inférieure vers le haut et la partie supérieure se déplaçant dans une direction sensiblement verticale.

Le corps creux peut être fermé, notamment hermétiquement fermé ou fermé de manière étanche, hormis au niveau du ou des orifices de sortie.

Le corps creux est de préférence au moins partiellement réalisé dans un matériau métallique, par exemple un acier inoxydable. Le corps creux peut présenter une paroi extérieure en acier inoxydable, ce matériau possédant une très bonne inertie thermique.

Le corps creux peut loger un tuyau intérieur configuré pour faire circuler les vapeurs, relié de manière étanche au bac et à l'orifice de sortie. Le tuyau intérieur est de préférence réalisé au moins partiellement dans au moins un matériau métallique, notamment en cuivre. Le tuyau intérieur comporte par exemple une pluralité de chambres successives formées chacune par une section agrandie du tuyau reliées entre elles par des sections rétrécies du tuyau. Un intérêt de cette succession de chambres est de créer un effet Venturi optimal pour une meilleure qualité de diffusion des vapeurs d'huiles essentielles.

L'intérêt de l'acier du corps creux peut être de jouer un rôle d'isolant permettant, lorsque présent, au cuivre du tuyau intérieur de conserver la chaleur à l'intérieur du diffuseur et de ne pas chauffer la surface du diffuseur, accessible à un utilisateur.

Le bac est de préférence indépendant du corps creux et avantageusement amovible du corps creux. Le corps creux peut alors comporter, dans la partie inférieure, une trappe d'accès pour accéder au moins au bac depuis l'extérieur du diffuseur. La trappe peut s'ouvrir par une simple pression, à l'aide d'un bouton poussoir. Dans la position fermée, la trappe peut être verrouillée à l'aide d'un organe de verrouillage, par exemple un barillet à clé. Le bac peut être muni d'une poignée, par exemple rétractable, pour permettre son retrait hors du diffuseur.

Le bac peut être réalisé de manière à être sensiblement isotherme, c'est-à-dire à permettre de maintenir au moins pendant un certain temps la chaleur du liquide qu'il contient. Le bac est ainsi par exemple réalisé en acier inoxydable présentant une double paroi avec un matériau isolant, par exemple une ouate de cellulose ou un feutre de laine, intercalé entre les deux parois. Ainsi, le bac peut maintenir au chaud un liquide pendant plusieurs heures à l'abri de l'air, donc sans qu'il soit contaminé par l'air ambiant. La double paroi permet également d'isoler la main d'un utilisateur tenant le bac de la chaleur du liquide contenu dans celui-ci.

Le bac peut avoir une capacité maximale comprise entre 50cl et 31, de préférence comprise entre 50 cl et 21, par exemple égale à 75cl ou 100cl.

Le bac peut être muni d'un bec verseur de manière à faciliter le versement du liquide qu'il contient dans un bol ou un verre si besoin.

Le liquide est de préférence choisi dans le groupe constitué par de l'eau, de l'huile végétale, de l'huile essentielle, de l'eau parfumée, un parfum d'ambiance, et un mélange de ceux-ci. Le liquide est de préférence de l'eau.

Ledit au moins un orifice de sortie peut être formé par une extrémité supérieure d'une cheminée fixée à la partie supérieure, notamment à une extrémité supérieure, du corps creux. Dans ce cas, la cheminée, qui forme un tuyau de sortie des vapeurs, est avantageusement montée pivotante et/ou orientable sur le corps creux de manière à pouvoir pivoter ou avoir une orientation modifiée par rapport au corps creux, lorsque manipulée, directement ou indirectement, par un utilisateur. Ainsi, son orientation par rapport au corps creux peut être modifiée de façon à orienter comme souhaité le jet de vapeurs sortant par l'orifice de sortie.

Le système de chauffage, de préférence réglable et/ou programmable et/ou régulé par exemple par un thermostat, est avantageusement disposé sous le bac, par exemple à une extrémité inférieure du corps creux ou sous l'extrémité inférieure du corps creux. Le système de chauffage est par exemple choisi dans le groupe constitué par une résistance électrique, une plaque chauffante électrique, à gaz, à induction, une batterie. Le système de chauffage peut être relié à un programmateur, par exemple à un thermostat, et à au moins un bouton de commande apte à permettre à un utilisateur de régler le programmateur.

Le bac est avantageusement indépendant du système de chauffage, notamment d'une partie électrique de celui-ci.

Le système de chauffage peut par exemple comporter une résistance électrique, pouvant être programmable et thermorégulatrice, insérée sous le bac. Le système de chauffage peut être logé dans le corps creux à une extrémité inférieure de celui-ci. En variante, le système de chauffage peut être positionné à part, sous l'extrémité inférieure du corps creux.

Le système de chauffage peut être prévu pour chauffer à plusieurs températures différentes prédéterminées, par exemple trois voire quatre températures différentes prédéterminées, ou encore davantage.

Le système de chauffage peut en particulier permettre le chauffage du liquide présent dans le bac à des températures de 45° C et 55° C, pour un cycle de diffusion, par exemple de 30 minutes. Le système de chauffage peut être prévu pour s'arrêter de chauffer dès que le liquide contenu dans le bac atteint 45°C ou 55°C et maintenir ces températures le temps du cycle de diffusion.

Le système de chauffage peut également permettre le chauffage du liquide présent dans le bac à la température de 80°C. Le système de chauffage peut être prévu pour s'arrêter de chauffer dès que le liquide atteint 80°C, le liquide, notamment l'eau étant alors dans un état dit frémissant. Le système de chauffage peut également permettre le chauffage du liquide présent dans le bac à la température de 100°C. Le système de chauffage peut être prévu pour s'arrêter de chauffer dès que le liquide, notamment l'eau, atteint cette température de 100°C, correspondant à la température d'ébullition de l'eau.

La température optimale de chauffe des huiles essentielles se situe entre 30°C et 60°C. Au-delà de 60°C, la préservation des propriétés et des arômes des huiles essentielles peut ne plus être aussi bien assurée. Grâce à l'invention, la diffusion peut être douce, à 45°C ou plus intense, grâce à un mode de chauffage à 55°C. Dans les deux cas, le chauffage ne dénaturera pas les qualités des huiles essentielles.

Le dispositif de régulation du débit de vapeurs peut comporter au moins une vanne réglable disposée sur le trajet des vapeurs dans le corps creux, la vanne étant agencée de manière à pouvoir occuper au moins :
- une position fermée dans laquelle la vanne empêche le passage de vapeurs, et
- une position ouverte dans laquelle la vanne permet le passage de vapeurs,
la position fermée ou ouverte étant avantageusement réglée à l'aide de l'organe de commande.

Le dispositif de régulation du débit de vapeurs peut par exemple comporter une vanne connue sous le nom de vanne papillon permettant de régler le débit de vapeurs circulant dans le corps creux, ou tout autre type de vanne, par exemple vanne à opercule, vanne à boisseau conique ou sphérique, vanne à plusieurs voies, vanne à guillotine, de manière non exhaustive. La vanne papillon comporte un disque appelé opercule, de diamètre sensiblement égal à un passage à obturer, pouvant pivoter autour d'un axe s'étendant selon un diamètre du disque pour ouvrir ou obturer totalement ou partiellement le passage. Le contrôle de l'angle de pivotement de l'opercule permet ainsi de régler le débit de vapeurs.

La vanne peut encore comporter plusieurs vantaux reliés entre eux et pivotants, notamment au nombre de cinq, agencés de manière à pouvoir occuper au moins une position fermée dans laquelle les vantaux obturent le passage de vapeurs, et une position ouverte dans laquelle les vantaux permettent le passage de vapeurs et au moins une position intermédiaire.

Lorsqu'il y a un tuyau intérieur présent à l'intérieur du corps creux, la vanne, notamment la vanne papillon, peut être présente de manière à pouvoir fermer intérieurement le tuyau empêchant alors la circulation de vapeurs, ou l'ouvrir partiellement ou totalement afin de laisser passer des vapeurs jusqu'à l'orifice de sortie.

La vanne, notamment la vanne papillon, peut être positionnée au-dessus du bac à une distance notamment comprise entre 5 cm et 30 cm, par exemple 9 cm au-dessus du bac.

L'organe de commande peut comporter une manette de réglage, manipulable par l'utilisateur, commandant la vanne. La vanne, notamment la vanne papillon, est alors reliée à la manette présente à l'extérieur du corps creux. Par exemple, en tournant la manette vers la droite, la vanne se ferme, empêchant la circulation de vapeurs. En la tournant vers la gauche, la vanne s'ouvre au maximum, permettant la circulation de vapeurs au débit maximum. Une ou plusieurs positions intermédiaires entre ces deux positions extrêmes peuvent être prévues, pour permettre la circulation de vapeurs à un débit intermédiaire. La fermeture hermétique de la vanne peut permettre de maintenir au chaud le liquide contenu dans le bac sans dépense d'énergie. La fermeture hermétique de la vanne peut encore permettre de préserver de la contamination de l'air ambiant un liquide chaud ou froid et d'éventuels substrats comportant des plantes au contact du liquide, les plantes pouvant rester toute une nuit au contact d'un liquide froid, notamment de l'eau, afin d'en extraire les principes actifs.

L'ouverture et la fermeture de la vanne peut, de manière alternative, être contrôlée par un organe de commande pneumatique, mécanique, électrique ou électronique, accessible pour l'utilisateur depuis un boîtier de commande.

Le diffuseur de vapeurs peut encore comporter un élément de chauffage additionnel, de préférence réglable et/ou programmable et/ou régulé par exemple par un thermostat, disposé le long du tuyau intérieur, à l'extérieur de celui-ci, avantageusement de façon continue, notamment en amont ou en aval de la vanne papillon. Un tel élément de chauffage permet de chauffer ou préserver la chaleur des vapeurs à l'intérieur du tuyau intérieur, ce qui peut améliorer leur qualité et faciliter leur remontée au travers du tuyau intérieur.

L'élément de chauffage additionnel peut également s'étendre au-dessus du bac, au moins partiellement autour d'un couvercle ouvert, notamment un entonnoir renversé, en forme conique, couvrant le bac et menant au tuyau intérieur. En particulier, l'élément de chauffage additionnel peut s'étendre jusqu'à la trappe d'accès afin de pouvoir chauffer l'ensemble de la partie du tuyau se trouvant juste au-dessus du bac. Pour cela, un té de dérivation peut être prévu, étant adapté à l'élément de chauffage et permettant à celui-ci de remonter le long du tuyau intérieur.

L'élément de chauffage additionnel peut être indépendant ou non du système de chauffage du liquide placé dans le bac. L'élément de chauffage additionnel peut être choisi dans le groupe constitué par un fil chauffant, un câble chauffant, un tuyau chauffant, une gaine chauffante, un ruban chauffant, une corde chauffante, un flexible chauffant, un anneau chauffant et une résistance chauffante. L'élément de chauffage additionnel peut être alimenté électriquement, à gaz, à induction ou par une batterie.

Le diffuseur de vapeurs peut encore comporter une gaine ou une mousse entourant au moins partiellement l'élément de chauffage additionnel, afin notamment de le protéger, une telle gaine ou mousse pouvant comporter un matériau par exemple polymère, notamment le polyamide, le polyéthylène réticulé, le polychlorure de vinyle (PVC), le silicone. Dans ce cas, l'élément de chauffage additionnel et la gaine ou mousse peuvent apporter une réponse rapide du chauffage, une isolation thermique et une sécurité de l'élément de chauffage avec faible déperdition de chaleur.

L'élément de chauffage pourra être fixé au tuyau intérieur notamment par sertissage, collage, adhérence, et/ou enroulage. Le diffuseur peut encore comporter, reliés à l'élément de chauffage additionnel, un programmateur, par exemple un thermostat, et au moins un bouton de commande apte à permettre à un utilisateur de régler le programmateur. L'utilisateur peut choisir par exemple de chauffer le tuyau intérieur à 45°C ou 55°C.

Au cas où l'élément de chauffage est dépendant du système de chauffage du bac, et lorsque le bac est chauffé à 45°C ou 55°C pour un cycle de diffusion, par exemple de 30 minutes, alors le tuyau intérieur pourra être chauffé en même temps *via* l'élément de chauffage additionnel.

Le diffuseur peut comporter en outre un panier amovible, notamment ajouré, agencé pour contenir un substrat, tel que des plantes ou parties de plantes, et prévu pour être disposé dans le bac en contact avec le liquide, le panier comportant de préférence des mailles serrées ou des orifices pour la diffusion d'une partie du substrat dans le liquide.

Le panier peut être en matériau métallique, par exemple en acier inoxydable, avec une maille serrée. On ne sort pas du cadre de l'invention si le panier n'est pas en métal, mais dans un autre matériau, par exemple au moins partiellement en papier, sous forme d'un filtre. Le panier peut comporter une pluralité d'orifices pour le passage des principes actifs des plantes qu'il peut contenir, par exemple entre 20 et 100 orifices de diamètre compris entre 0,5 mm et 5 mm, de préférence entre 1 mm et 2 mm, par exemple 49 orifices de 1,9 mm de diamètre.

Le panier peut être équipé d'un manche, par exemple amovible, de manière à faciliter sa manipulation en toute sécurité.

Le panier peut être logé au moins partiellement dans le bac. Il peut être prévu pour se placer à hauteur intermédiaire, notamment à mi-hauteur, dans le bac à l'aide de supports, par exemple au nombre de quatre, disposés, étant fixés ou rapportés, sur la paroi intérieure du bac. Lorsque le panier est réalisé au moins partiellement en papier, il peut comporter une pièce rigide fixée au papier et permettant le positionnement du panier sur des supports.

Le panier peut contenir des plantes ou parties de plantes pour réaliser des infusions ou décoctions ou macérations au contact avec le liquide présent dans le bac. En effet, la plante ou les parties de plantes ou substrat contenus dans le panier peuvent libérer leurs principes actifs à travers les orifices du panier et se retrouver dans la moitié inférieure du bac, dans le liquide. Bien entendu, selon l'usage du diffuseur, le panier peut être ou ne pas être utilisé, et forme un accessoire de celui-ci.

### Procédé de diffusion de vapeurs d'huiles essentielles

La présente invention a encore pour objet, selon un autre de ses aspects, en combinaison avec ce qui précède, un procédé de diffusion de vapeurs d'huiles essentielles à l'aide du diffuseur tel que défini plus haut, comportant les étapes suivantes :
- mettre un liquide dans le bac,
- ajouter des gouttes d'huiles essentielles dans le liquide, et
- faire chauffer le liquide contenu dans le bac à l'aide du système de chauffage, notamment à une température prédéterminée pendant une durée prédéterminée.

### Procédé de réalisation d'une infusion et/ou d'une décoction et/ou d'une macération

La présente invention a encore pour objet, en combinaison avec ce qui précède, selon un autre de ses aspects, un procédé de réalisation d'une infusion, à l'aide du diffuseur tel que défini plus haut, comportant les étapes suivantes :
- mettre un liquide dans le bac,
- faire chauffer le liquide à l'aide du système de chauffage, notamment à une température prédéterminée pendant une durée prédéterminée,
- disposer un substrat dans ou au contact du liquide chauffé du bac, le substrat étant de préférence contenu dans un panier ajouré disposé au contact du liquide chauffé,
- laisser infuser, par exemple pendant une durée comprise entre 1 et 10 min environ, qui peut être programmée ou non, afin d'obtenir une infusion.

La présente invention a encore pour objet, en combinaison avec ce qui précède, selon un autre de ses aspects, un procédé de réalisation d'une décoction, à l'aide du diffuseur tel que défini plus haut, comportant les étapes suivantes :
- mettre un liquide dans le bac,
- disposer un substrat dans ou au contact du liquide du bac, le substrat étant de préférence contenu dans un panier ajouré disposé au contact du liquide,
- faire chauffer le liquide à l'aide du système de chauffage, notamment à une température prédéterminée pendant une durée prédéterminée, afin d'obtenir une décoction.

La présente invention a encore pour objet, en combinaison avec ce qui précède, selon un autre de ses aspects, un procédé de réalisation d'une macération, à l'aide du diffuseur tel que défini plus haut, comportant les étapes suivantes :
- mettre un liquide dans le bac,
- disposer un substrat dans ou au contact du liquide du bac, le substrat étant de préférence contenu dans un panier ajouré disposé au contact du liquide,
- laisser reposer à froid avec le dispositif de réglage du débit de vapeurs dans une position fermée, étanche, pendant une durée prédéterminée, notamment une nuit entière, afin d'obtenir une macération.

Il peut n'y avoir aucun chauffage supplémentaire, une fois que le substrat a été introduit au contact du liquide chaud. En variante, il peut y avoir un chauffage complémentaire, par exemple sous forme de réchauffage. Le substrat est avantageusement choisi parmi les plantes et les parties de plantes, telles que feuilles, fleurs, sommités fleuries, racines, baies, graines, rameaux, écorces, bois.

Dans la mise en oeuvre de ces procédés, il est à noter que l'ensemble des fonctionnalités du diffuseur ne sont pas utilisées. Ainsi, pour la macération, on n'utilise pas le système de chauffage. Pour la réalisation d'une infusion, on peut ne pas utiliser le dispositif de régulation du débit de vapeurs.

Il est également à noter que le liquide peut ne pas contenir d'huiles essentielles. Dans ce cas, il est avantageusement mis au contact d'un substrat, notamment un substrat végétal, afin de modifier le liquide.

Pour mettre le liquide et/ou des huiles essentielles dans le bac, il peut être nécessaire de retirer le bac du corps creux. Le bac sera alors ensuite remis en place pour l'utilisation du diffuseur.

### Procédé de réalisation d'un liquide à destination d'une inhalation

La présente invention a encore pour objet, selon un autre de ses aspects, en combinaison avec ce qui précède, un procédé de réalisation d'un liquide à destination d'une inhalation à l'aide du diffuseur tel que défini plus haut, comportant les étapes suivantes :
- mettre un liquide dans le bac,
- faire chauffer le liquide à l'aide du système de chauffage, notamment à une température prédéterminée pendant une durée prédéterminée,
- retirer le bac du diffuseur,
- ajouter des gouttes d'huiles essentielles dans le liquide du bac,
- utiliser le liquide ainsi modifié pour réaliser une inhalation.

### Brève description des figures

L'invention pourra être mieux comprise à la lecture de la description qui va suivre, d'exemples de mise en oeuvre non limitatifs de celle-ci et à l'examen du dessin annexé, sur lequel :
- la figure 1 est une vue schématique en coupe axiale d'un exemple de diffuseur de vapeurs d'huiles essentielles conforme à l'invention,
- la figure 2 illustre de manière schématique en coupe axiale le bac du diffuseur de la figure 1 rempli de liquide et dans lequel on peut verser des gouttes d'huiles essentielles,
- la figure 3 représente isolément de manière schématique un autre exemple de bac, vu en perspective,
- la figure 4 représente isolément, de manière schématique et en coupe axiale, un panier ajouré pouvant être disposé sur le bac,
- la figure 5 représente isolément un autre exemple de panier ajouré, de manière schématique et en perspective,
- la figure 6 représente de manière schématique et isolément les touches de programmation et les voyants lumineux pour la commande du diffuseur,
- la figure 7 représente de manière schématique et isolément, vue de face, un exemple de trappe d'accès,
- la figure 8 illustre un autre exemple de diffuseur conforme à l'invention,
- la figure 9 est une vue similaire à la figure 1 d'un autre exemple de diffuseur conforme à l'invention,
- la figure 10 est une vue agrandie, schématique, d'un détail de la figure 9,
- la figure 11 représente schématiquement et partiellement, isolément, l'élément de chauffage additionnel de la figure 10, et
- la figure 12 représente schématiquement et isolément un bouton de commande de l'élément de chauffage additionnel.

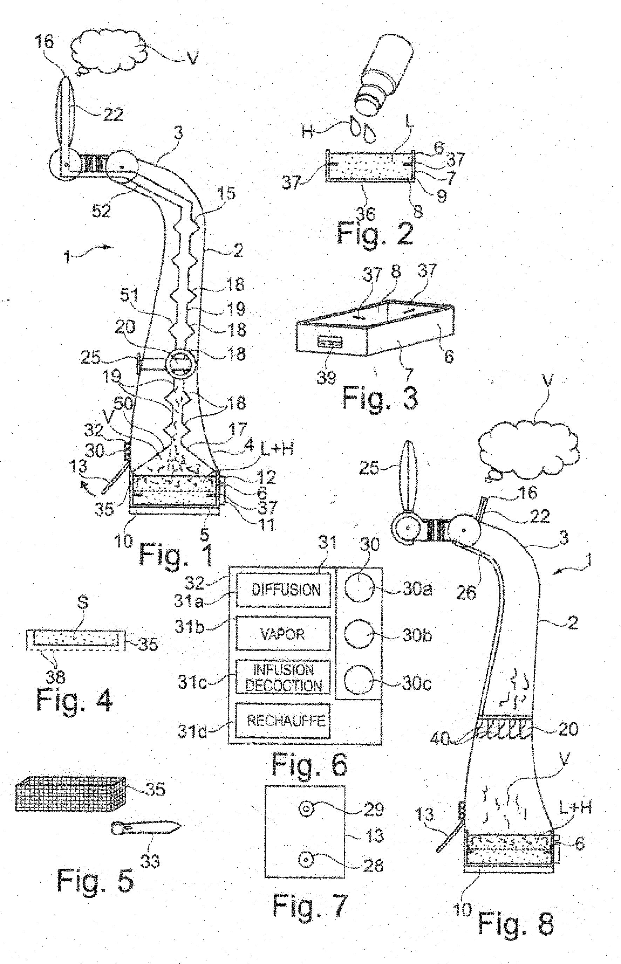

On a représenté sur la figure 1 un premier exemple de diffuseur 1 de vapeurs d'huiles essentielles, dans cet exemple par chaleur douce, conforme à l'invention.

### Description détaillée de modes de réalisation

Le diffuseur 1 comprend un corps creux 2, fermé, se présentant sous forme de colonne verticale, avec une partie supérieure 3 et une partie inférieure 4. La colonne formée par le corps creux 2 est incurvée dans sa partie supérieure 3, sensiblement en forme de S, comme visible. L'extrémité inférieure 5 du corps creux 2 est sensiblement plane et agencée pour reposer sur un support plan tel qu'une table ou le sol par exemple. Le corps creux 2 est avantageusement réalisé en acier inoxydable.

Le diffuseur 1 comporte en outre un bac 6, amovible, disposé dans la partie inférieure 4 du corps creux 2. Le bac 6 est destiné à contenir au moins un liquide L, de préférence de l'eau. Le liquide L, par exemple de l'eau, est additionné de gouttes d'huiles essentielles H comme visible sur la figure 2. Le bac 6 se présente sous forme d'un récipient présentant une double paroi, avec une paroi interne 8 et une paroi externe 7, réalisées en acier inoxydable. Dans l'espace séparant les deux parois 7 et 8 se trouve un matériau isolant 9 tel qu'un feutre de laine ou une ouate de cellulose. Le bac 6 peut être pourvu, comme visible dans le mode de réalisation de la figure 3, d'une poignée rétractable 39 ou amovible afin de le manipuler en toute sécurité. Le bac 6 peut être également muni, de manière non illustrée, d'un bec verseur afin de faciliter le versement du liquide dans un bol ou une tasse.

Le diffuseur 1 comporte en outre un système de chauffage 10 disposé dans le corps creux 2 dans la partie inférieure 4 de celui-ci. Le système de chauffage 10 est prévu pour chauffer le liquide L présent dans le bac afin de générer des vapeurs. Le système de chauffage 10 est disposé sous le bac 6 dans l'exemple illustré, de manière indépendante de celui-ci. Le système de chauffage 10 comporte par exemple une résistance électrique. En variante, sans sortir du cadre de l'invention, le système de chauffage 10 peut consister en une plaque électrique, à gaz ou à induction ou autre système de chauffage et/ou être disposé hors du corps creux, sous celui-ci, formant un socle.

Pour alimenter en électricité le système de chauffage 10, une connexion 11 pour recevoir une alimentation électrique par câble est prévue en partie inférieure 4 du corps creux 2, à l'extérieur de celui-ci comme visible. Si le système de chauffage est disposé hors du corps creux, la connexion ou le câble électrique peut alors être relié(e) au système de chauffage et non au corps creux. Il est à noter qu'on ne sort pas du cadre de l'invention si l'alimentation provient d'une ou plusieurs piles ou d'une batterie. Dans ce cas, aucune connexion 11 n'est nécessaire.

Un bouton de commande 12 On/Off permet de mettre le diffuseur 11 sous tension ou hors tension.

Dans l'exemple illustré, le diffuseur 1 comporte en outre en partie inférieure 4 du corps creux 2, une trappe d'accès 13 consistant en un battant pouvant pivoter entre une position ouverte et une position fermée. Un simple bouton poussoir 28, visible sur la figure 7, peut permettre d'ouvrir ou de fermer la trappe d'accès 13. En l'ouvrant, c'est-à-dire en pivotant dans le sens de la flèche illustrée sur la figure 1, vers le haut, cette trappe 13 peut être ouverte et un utilisateur peut accéder au bac 6 afin de le retirer pour, par exemple, comme illustré sur la figure 2, le remplir de liquide L et de gouttes d'huiles essentielles H. Un système de verrouillage 29, illustré sur la figure 7, consistant dans cet exemple en un système de barillet à clé, peut permettre d'empêcher l'ouverture de la trappe d'accès 13 si nécessaire. Un système de sécurité peut également être prévu pour prévenir des brûlures en cas de liquide ou de vapeurs trop chauds. Par exemple, lors du retrait du bac 6, un système de volet roulant, par exemple réalisé en acier inoxydable, non illustré dans un souci de clarté du dessin, vient refermer celui-ci à la manière d'un couvercle. Un tel volet roulant peut être adossé à la paroi 7 du bac ou être intégré dans l'espace séparant les parois 7 et 8. Le volet roulant pourra par exemple s'ouvrir grâce à une poignée située à son extrémité. Lorsque le bac 6 est remis en position dans la partie inférieure 4 du corps creux 2, le volet roulant sera en position ouverte.

Dans l'exemple illustré, le corps creux 2 loge intérieurement un tuyau intérieur 15 suivant la forme générale intérieure du corps creux 2, à distance des parois de celui-ci. Le tuyau intérieur 15 est configuré pour faire circuler les vapeurs depuis le bas vers le haut du diffuseur et se termine par un orifice de sortie 16 autorisant la sortie des vapeurs V.

Dans l'exemple illustré, le tuyau intérieur 15 forme, dans sa partie inférieure 50, un entonnoir 17 positionné tête en bas de manière étanche au-dessus du bac 6 pour recueillir un concentré de vapeurs V émis par le chauffage du liquide L.

Puis le tuyau intérieur 15, dans une partie intermédiaire 51 verticale, comporte une pluralité de chambres 18 de sections agrandies reliées entre elles par des sections 19 rétrécies. Le nombre de chambres 18 dans l'exemple illustré est de sept mais ce nombre peut être modifié sans sortir du cadre de l'invention. La forme spécifique du tuyau intérieur 15 avec les zones rétrécies 19 et les zones agrandies 18 permet aux vapeurs d'huiles essentielles d'accélérer au fur et à mesure de leur montée dans le tuyau et de leur passage successif à travers les rétrécissements, ce qui a pour effet d'augmenter le débit de vapeurs selon un effet Venturi, afin de permettre au diffuseur 1 d'améliorer son tirage pour mieux diffuser les vapeurs V dans l'espace extérieur. L'une de ces chambres 18 loge un dispositif de régulation du débit de vapeurs 20 qui sera décrit plus en détail ci-après. La forme des chambres 18 peut être telle qu'illustrée, en forme de double entonnoir reliés par leur section de plus grand diamètre. Un autre intérêt de la forme en double entonnoir est de bien séparer les particules d'huiles essentielles entre elles pour une meilleure diffusion. Une autre forme de chambres peut être, par exemple, une forme sensiblement de sphère.

Le tuyau intérieur 15 comporte enfin une section supérieure 52 suivant la courbure du corps creux 2 en partie supérieure 3 de celui-ci jusqu'à se terminer par une cheminée 22 débouchant sur l'orifice de sortie 16.

Les vapeurs V suivent le trajet partant du bac 6 jusqu'à l'orifice de sortie 16 à l'intérieur du tuyau intérieur 15.

Le tuyau intérieur 15 est avantageusement réalisé en cuivre qui présente les propriétés d'être un excellent conducteur thermique résistant à la corrosion et aux huiles essentielles et approprié pour une utilisation avec l'eau. Le cuivre est également un puissant bactéricide et fongicide.

Le corps creux 2 en acier inoxydable permet de conférer une très bonne inertie thermique au diffuseur 1, ce qui permet de jouer un rôle d'isolant à la surface extérieure du corps creux 2 afin d'éviter les brûlures d'un utilisateur et de maintenir une température suffisante à l'intérieur du diffuseur 1.

Le diffuseur 1 comporte comme indiqué ci-dessus un dispositif 20 de régulation du débit de vapeurs pour la régulation des vapeurs V s'échappant par l'orifice de sortie 16. Ce dispositif 20 est disposé dans le corps creux 2 entre le bac 6 et l'orifice de sortie 16. Dans cet exemple, le dispositif de régulation du débit de vapeurs 20 est disposé, comme indiqué ci-dessus, dans une chambre 18, la troisième en partant du bas comme illustré, à l'intérieur du tuyau intérieur 15.

Dans l'exemple illustré, ce dispositif 20 de régulation du débit de vapeurs comporte une vanne réglable disposée sur le trajet des vapeurs dans le corps creux, cette vanne étant agencée de manière à pouvoir occuper au moins une position fermée dans laquelle la vanne empêche le passage de vapeurs et une position ouverte dans laquelle la vanne permet le passage de vapeurs. La position fermée ou ouverte est réglée à l'aide de l'organe de commande 25 consistant dans cet exemple en une manette de réglage. La manette de réglage est présente à l'extérieur du diffuseur 1 pour être accessible à un utilisateur et reliée à la vanne.

Le dispositif de régulation du débit de vapeurs 20 comporte dans cet exemple une vanne de type vanne papillon. Outre une position ouverte maximale et une position fermée, l'organe de commande 25 peut permettre de régler le dispositif 20 de régulation du débit de vapeurs dans au moins une position intermédiaire légèrement ouverte.

Des voyants 30 lumineux et touches 31 de programmation d'un programmateur 32, présent sur une surface extérieure du corps creux 2, illustrés de manière isolée sur la figure 6 sont prévus pour permettre de choisir un programme ou donner une information à l'utilisateur, par exemple :
- une touche 31a intitulée « Diffusion » pour une diffusion douce à 45°C,
- une touche 31b intitulée « VapoR » pour une diffusion plus intense à 55°C,
- une touche 31c intitulée «Infusion-Décoction» pour un chauffage du liquide L à 80°C ou 100°C,
- une touche 31d intitulée « Réchauffe » pour un chauffage du liquide L à 55°C,
- un voyant lumineux 30a par exemple de couleur verte indiquant que le mode « Diffusion » ou « VapoR » est en marche ou que le liquide est chauffé à 45°C ou 55°C ou 80°C ou 100°C,
- un voyant lumineux 30b par exemple de couleur rouge, indiquant que le système de chauffage 10 est en marche et le liquide L en train de chauffer, pour une température finale à 45°C ou 55°C ou 80°C ou 100°C,
- un voyant lumineux 30c par exemple de couleur orange, pour indiquer que le liquide L est chaud et maintenu à une température de 55°C par exemple.

Un panier 35 amovible, ajouré, avantageusement métallique, de préférence en acier inoxydable, illustré de manière isolée sur la figure 4, comportant des orifices 38, au nombre par exemple de quarante-neuf, présentant chacun un diamètre par exemple de 1,9 mm, peut être positionné dans le bac 6 au contact du liquide L et contenir en lui-même des plantes ou des parties de plantes formant un substrat S. Le panier 35 peut être maintenu à distance du fond 36 du bac 6 à l'aide de supports 37, au nombre de deux ou quatre, par exemple présents sur la paroi 8 intérieure du bac 6 ou sous le panier 35, comme visible notamment sur la figure 3.

Un autre exemple de panier 35 ajouré, illustré sur la figure 5, forme un panier grillagé à mailles serrées, pourvu d'une poignée amovible 33.

Grâce au diffuseur 1, on peut diffuser des vapeurs d'huiles essentielles en procédant comme suit. Tout d'abord, on retire, en ouvrant la trappe 13, le bac 6 du diffuseur 1. On ajoute un liquide L, par exemple de l'eau, et des gouttes d'huiles essentielles H. Après avoir remis le bac 6 dans le diffuseur 1 et fermé la trappe 13, on allume l'interrupteur 12, le diffuseur 1 étant connecté à une prise électrique.

L'utilisateur choisit par exemple un programme à l'aide du programmateur 32. Il peut choisir de chauffer à 45°C ou 55°C pour un cycle de diffusion par exemple de 30 minutes. Pour les températures de 45°C ou 55°C, le système de chauffage 10 peut être programmé pour maintenir la température pendant tout le temps du cycle de diffusion.

Lorsque le liquide L est chauffé, des vapeurs V additionnées d'huiles essentielles sont créées, lesquelles se diffusent à l'intérieur du tuyau intérieur 15 jusqu'à l'orifice de sortie 16 et elles sortent, comme illustré, pour se diffuser dans l'espace situé autour du diffuseur 1.

Outre la diffusion de vapeurs d'huiles essentielles à l'aide du diffuseur 1, on peut également utiliser le diffuseur 1 pour réaliser des décoctions en mettant un liquide L dans le bac 6, en disposant les plantes ou parties de plantes dans ou au contact du liquide L du bac 6, les plantes ou parties de plantes étant de préférence contenues dans le panier 35 ajouré disposé au contact du liquide. Puis on fait chauffer, à l'aide du système de chauffage 10, le liquide L, notamment à une température prédéterminée pendant une durée prédéterminée.

Pour réaliser une infusion, on chauffe d'abord le liquide L, jusqu'à ce que sa température atteigne la température prédéterminée, à l'aide du système de chauffage. Alors, les plantes ou parties de plantes sont mises dans ou au contact du liquide L du bac 6, les plantes ou parties de plantes étant de préférence contenues dans le panier 35 ajouré disposé au contact du liquide L. Puis, on laisse infuser pendant une durée par exemple comprise entre 1 et 10 min environ, afin d'obtenir une infusion. On ne sort pas, bien entendu, du cadre de l'invention si la durée d'infusion est supérieure à 10 min.

Pour réaliser une macération, on dispose les plantes ou les parties de plantes dans ou au contact du liquide L du bac 6, étant de préférence contenues dans le panier 35 ajouré, et on laisse reposer à froid et de façon hermétique, en mettant le dispositif de réglage du débit de vapeurs 20 dans sa position fermée, pendant par exemple une nuit entière.

Pour finir, on utilise le liquide L ainsi modifié pour l'infusion, la décoction et/ou la macération, en ouvrant la trappe 13 et en retirant le bac 6.

Enfin, on peut utiliser le diffuseur 1 selon l'invention pour réaliser un liquide à destination d'une inhalation en procédant comme pour la réalisation d'une infusion mais en utilisant le liquide L chauffé jusqu'à ce que sa température atteigne la température prédéterminée, à l'aide du système de chauffage 10 puis en ajoutant quelques gouttes d'huiles essentielles dans le liquide L sorti du diffuseur 1.

La figure 8 illustre un autre exemple de réalisation d'un diffuseur 1 conforme à l'invention. Dans cet exemple, le corps creux 2 ne contient pas de tuyau intérieur 15 mais forme en lui-même le contenant des vapeurs qui vont s'élever depuis le bac 6 jusqu'à l'orifice de sortie 16 lorsque l'on chauffe le liquide L contenu dans le bac 6 à l'aide du système de chauffage 10. La forme de cet autre exemple de diffuseur est telle qu'elle permet aux vapeurs d'huiles essentielles d'accélérer au fur et à mesure de leur montée dans le contenant et de leur passage successif à travers les rétrécissements et élargissements du diffuseur, avec le diffuseur par exemple en forme de S. Cela a pour effet d'augmenter le débit de vapeurs selon un phénomène de type Venturi, afin de permettre au diffuseur d'améliorer son tirage pour mieux diffuser les vapeurs V dans l'espace extérieur.

Une autre différence consiste dans le dispositif 20 de régulation du débit de vapeurs constitué dans cet exemple par une pluralité de vantaux 40 permettant d'ouvrir ou de fermer le débit de vapeurs. Dans l'exemple de la figure 8, la manette de réglage 25 se situe en partie supérieure 3 du corps creux 2 et sa manipulation permet via un câble 26 d'orienter les vantaux 40 pour obtenir le degré d'ouverture souhaité ou la fermeture hermétique des vantaux afin d'empêcher le passage de vapeurs. Enfin, la cheminée 22 est située à un autre endroit du mode de réalisation illustré sur la figure 1, mais toujours en partie supérieure 3 du corps creux 2. Cette cheminée 22 est montée pivotante sur le corps creux 2, étant orientable comme dans l'exemple de la figure 1 afin de diffuser les vapeurs V dans la direction souhaitée par l'utilisateur.

L'invention n'est bien entendu pas limitée aux exemples qui viennent d'être décrits.

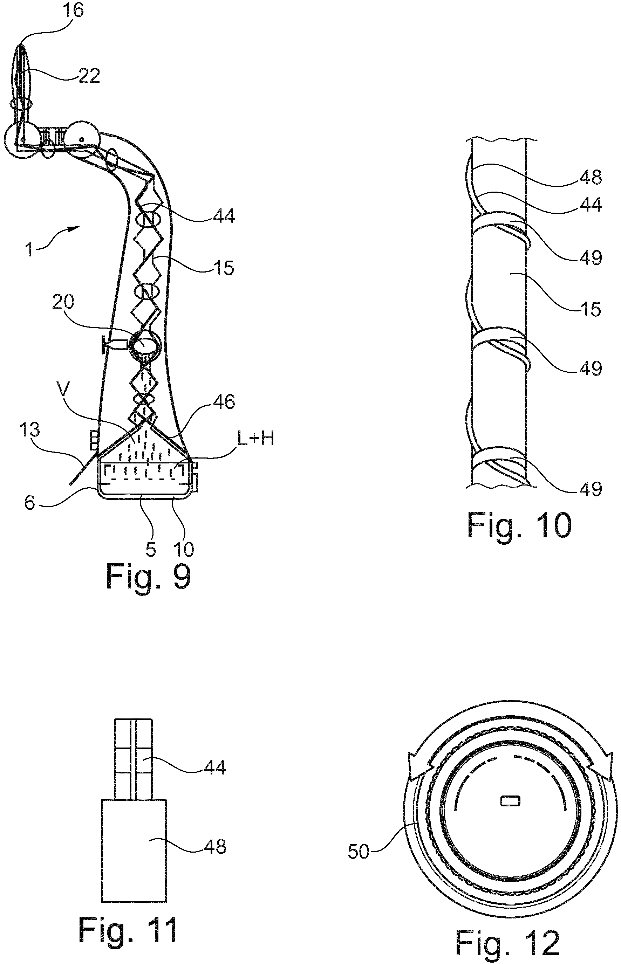

En particulier, le diffuseur de vapeurs 1 peut intégrer, comme dans le mode de réalisation illustré sur les figures 9 à 12, un élément de chauffage additionnel 44, disposé le long du tuyau intérieur 15, à l'extérieur de celui-ci comme illustré sur la figure 9.

Dans l'exemple illustré, l'élément de chauffage additionnel 44 entoure de façon continue le tuyau intérieur 15, étant enroulé autour de celui-ci, en amont ou en aval de la vanne papillon 20 et également s'étend au-dessus du bac 6, au moins partiellement autour du couvercle 46 ouvert, notamment en forme conique en entonnoir renversé, couvrant le bac 6 et menant au tuyau intérieur 15. En particulier, l'élément de chauffage additionnel 44 peut s'étendre jusqu'à la trappe d'accès 13 afin de pouvoir chauffer l'ensemble de la partie du tuyau 15 se trouvant juste au-dessus du bac 6.

Dans l'exemple illustré, comme visible sur les figures 9 et 10, l'élément de chauffage additionnel 44 forme un câble chauffant. L'élément de chauffage additionnel 44 peut être alimenté électriquement, à gaz, à induction ou par une batterie (non visible sur les figures dans un souci de clarté du dessin). Comme visible en particulier sur la figure 11, une gaine 48, extérieure, entoure l'élément de chauffage additionnel 44, afin notamment de le protéger. Dans l'exemple illustré, la gaine 48 comporte un polyamide.

Toujours dans l'exemple illustré, l'élément de chauffage 44 est fixé au tuyau intérieur 15 à l'aide de colliers de serrage 49, comme visible sur la figure 10.
L'élément de chauffage additionnel 44 peut être indépendant du système de chauffage 10. Dans l'exemple illustré, le diffuseur 1 comporte, reliés à l'élément de chauffage additionnel 44, un programmateur, par exemple un thermostat, et au moins un bouton de commande 50, visible sur la figure 12, apte à permettre à un utilisateur de régler le programmateur. L'utilisateur peut choisir par exemple de chauffer le tuyau intérieur 15 à 45°C ou 55°C à l'aide de l'élément de chauffage additionnel 44. On ne sort pas du cadre de l'invention si l'élément de chauffage additionnel 44 est commandé de manière dépendante du système de chauffage 10, et notamment du programmateur 32.

## Revendications

1. Diffuseur (1) de vapeurs, notamment d'huiles essentielles, comportant :
- un corps creux (2),
- un bac (6) pour contenir au moins un liquide (L) et éventuellement des huiles essentielles (H), logé dans une partie inférieure (4) du corps creux (2),
- un système de chauffage (10) pour chauffer le liquide (L) présent dans le bac afin de créer des vapeurs (V),
- au moins un orifice de sortie (16) des vapeurs réalisé dans une partie supérieure (3) du corps creux (2),
- un dispositif de régulation du débit de vapeurs (20) s'échappant par l'orifice de sortie (16), disposé dans le corps creux (2) entre le bac (6) et l'orifice de sortie (16), et
- un organe de commande (25) dudit dispositif de régulation du débit de vapeurs (20).

2. Diffuseur selon la revendication 1, dans lequel le corps creux (2) loge un tuyau intérieur (15) configuré pour faire circuler les vapeurs, relié de manière étanche au bac (6) et à l'orifice de sortie (16), ledit tuyau (15) étant réalisé au moins partiellement dans au moins un matériau métallique, notamment en cuivre.

3. Diffuseur selon la revendication précédente, le tuyau intérieur (15) comportant une pluralité de chambres (18) successives formées chacune par une section agrandie du tuyau reliées entre elles par des sections rétrécies (19) du tuyau.

4. Diffuseur selon l'une quelconque des revendications précédentes, dans lequel le système de chauffage (10), de préférence réglable et/ou programmable et/ou régulé par un thermostat, est disposé sous le bac (6), et est choisi dans le groupe constitué par une résistance électrique, une plaque chauffante électrique, à gaz, à induction, une batterie.

5. Diffuseur selon l'une quelconque des revendications précédentes, dans lequel le dispositif de régulation du débit de vapeurs (20) comporte au moins une vanne réglable disposée sur le trajet des vapeurs dans le corps creux, la vanne étant agencée de manière à pouvoir occuper au moins :
- une position fermée dans laquelle la vanne empêche le passage de vapeurs, et
- une position ouverte dans laquelle la vanne permet le passage de vapeurs,
la position fermée ou ouverte étant réglée à l'aide de l'organe de commande (25).

6. Diffuseur selon la revendication précédente, dans lequel l'organe de commande (25) comporte une manette de réglage, manipulable par l'utilisateur, commandant ladite vanne.

7. Diffuseur selon l'une quelconque des revendications précédentes, comportant en outre un panier (35) amovible, notamment ajouré, agencé pour contenir un substrat (S), tel que des plantes ou parties de plantes, et prévu pour être disposé dans le bac (6) en contact avec le liquide (L), le panier comportant de préférence des mailles serrées ou des orifices pour la diffusion d'une partie du substrat dans le liquide.

8. Diffuseur selon l'une quelconque des revendications précédentes, comportant un élément de chauffage additionnel, de préférence réglable et/ou programmable et/ou régulé par exemple par un thermostat, disposé le long du tuyau intérieur, à l'extérieur de celui-ci, fixé au tuyau intérieur notamment par sertissage, collage, adhérence, et/ou enroulage.

9. Diffuseur selon la revendication précédente, dans lequel l'élément de chauffage additionnel peut être choisi dans le groupe constitué par un fil chauffant, un câble chauffant, un tuyau chauffant, une gaine chauffante, un ruban chauffant, une corde chauffante, un flexible chauffant, un anneau chauffant et une résistance chauffante.

10. Diffuseur selon la revendication 8 ou 9, comportant une gaine ou une mousse entourant au moins partiellement l'élément de chauffage additionnel, une telle gaine ou mousse comportant un matériau par exemple polymère, notamment le polyamide, le polyéthylène réticulé, le polychlorure de vinyle (PVC), le silicone.

11. Procédé de diffusion de vapeurs d'huiles essentielles, à l'aide du diffuseur (1) selon l'une quelconque des revendications 1 à 10, comportant les étapes suivantes :
- mettre un liquide (L) dans le bac (6),
- ajouter des gouttes d'huiles essentielles (H) dans le liquide (L), et
- faire chauffer le liquide (L) contenu dans le bac (6) à l'aide du système de chauffage (10), notamment à une température prédéterminée pendant une durée prédéterminée.

12. Procédé de réalisation d'une infusion à l'aide du diffuseur (1) selon l'une quelconque des revendications 1 à 10, comportant les étapes suivantes :
- mettre un liquide (L) dans le bac (6),
- faire chauffer à l'aide du système de chauffage (10), notamment à une température prédéterminée pendant une durée prédéterminée,
- disposer un substrat (S) dans ou au contact du liquide du bac (6), le substrat (S) étant de préférence contenu dans un panier (35) ajouré disposé au contact du liquide,
- laisser infuser, notamment pendant une durée comprise entre 1 min et 10 min, qui peut être programmée ou non, afin d'obtenir une infusion.

13. Procédé de réalisation d'une décoction à l'aide du diffuseur (1) selon l'une quelconque des revendications 1 à 10, comportant les étapes suivantes :
- mettre un liquide (L) dans le bac (6),
- disposer un substrat (S) dans ou au contact du liquide du bac (6), le substrat (S) étant de préférence contenu dans un panier (35) ajouré disposé au contact du liquide,
- faire chauffer à l'aide du système de chauffage (10), notamment à une température prédéterminée pendant une durée prédéterminée, afin d'obtenir une décoction.

14. Procédé de réalisation d'une macération à l'aide du diffuseur (1) selon l'une quelconque des revendications 1 à 10, comportant les étapes suivantes :
- mettre un liquide (L) dans le bac (6),
- disposer un substrat (S) dans ou au contact du liquide du bac (6), le substrat (S) étant de préférence contenu dans un panier (35) ajouré disposé au contact du liquide,
- laisser reposer à froid, le dispositif de régulation du débit de vapeurs étant dans une position fermée, étanche, pendant une durée prédéterminée, notamment une nuit entière, afin d'obtenir une macération.

15. Procédé de réalisation d'un liquide à destination d'une inhalation à l'aide du diffuseur (1) selon l'une quelconque des revendications 1 à 10, comportant les étapes suivantes :
- mettre un liquide (L) dans le bac (6),
- faire chauffer le liquide à l'aide du système de chauffage (10), notamment à une température prédéterminée pendant une durée prédéterminée,
- retirer le bac (6) du diffuseur (1),
- ajouter des gouttes d'huiles essentielles dans le liquide (L)
- utiliser le liquide (L) ainsi modifié pour réaliser une inhalation.
